Europäisches Patentamt

European Patent Office

Office européen des brevets

(11) Publication number: **0 299 504**
**A1**

## EUROPEAN PATENT APPLICATION

(21) Application number: 88111398.9

(22) Date of filing: 15.07.88

(51) Int. Cl.⁴: **C07D 231/12**

(30) Priority: 17.07.87 CS 5446/87

(43) Date of publication of application:
18.01.89 Bulletin 89/03

(84) Designated Contracting States:
**AT CH DE FR GB IT LI SE**

(71) Applicant: **SPOFA spojené podniky pro zdravotnickou vyrobu**
**No 11a Husinecká**
**Praha 3(CS)**

(72) Inventor: **Hajicek, Josef**
**Lumirova 15**
**Praha(CS)**
Inventor: **Pihera, Pavel**
**Limuzska 530**
**Praha(CS)**
Inventor: **Brunova, Bohumila**
**Ostrovského 33**
**Praha(CS)**
Inventor: **Michalsky, Jiri, RNDr.**
**Foersterova 45**
**Olomouc(CS)**
Inventor: **Hrbata, Jiri, Dr.**
**Josefa Hory 8**
**Prostejov(CS)**
Inventor: **Ferenc, Milan, RNDr.**
**J. Heyrovského 35**
**Olomouc(CS)**

(74) Representative: **Patentanwälte Beetz sen. - Beetz jun. Timpe - Siegfried - Schmitt-Fumian- Mayr**
**Steinsdorfstrasse 10**
**D-8000 München 22(DE)**

(54) A process for the preparation of 1-phenyl-3-(4-chlorophenyl)-pyrazol-4-ylacetic acid and its calcium salt.

(57) The invention relates to a process for preparing 1-phenyl-3-(4-chlorophenyl)-pyrazol-4-ylacetic acid, known under the generic name Lonazolac, and its calcium salt (Ca-Lonazolac). The process comprises the reductive hydrolysis of 1-phenyl-3-(4-chlorophenyl)-4-pyrazolecarboxaldehyde cyanohydrine using stannous chloride in a mixture of acetic acid, hydrochloric acid and preferably a catalytic amount of hydroiodic acid at elevated temperatures, and, if required, subsequent conversion of the acid into the calcium salt. The products are potent anti-inflammatory agents.

# A process for the preparation of 1-phenyl-3-(4-chlorophenyl)-pyrazol-4-ylacetic acid and its calcium salt

The invention relates to a process for the production of 1-phenyl-3-(4-chlorophenyl)-pyrazol-4-ylacetic acid and its calcium salt.

The subject acid (Lonazolac, generic name) and its calcium salt have high anti-inflammatory activity and besides are potent inhibitors of thrombocyte aggregation. A known multistage preparation method involves reaction of the respective 1,3-diaryl-4-pyrazolecarboxaldehyde (available by cyclization of the corresponding 4-substituted acetophenone phenylhydrazone using phosphoryl chloride in dimethylformamide) with expensive hydride reducing agents, e.g. sodium borohydride, subsequent conversion of the formed alcohol via the corresponding chloride to the respective nitrile using successively thionyl chloride and sodium cyanide, and final acidic or alkaline hydrolysis at elevated temperatures. Another known method for the preparation of said acid, its calcium salt and related compounds consists in hydroxymethylation of 3-aroylpropionic acids with formaldehyde, cyclization of the hydroxymethyl intermediates to aroylbutanolides, prolonged heating of the product with phenylhydrazine and oxidation of the resulting substituted dihydropyrazolylacetic acid, optionally after previos conversion of the acid into its calcium salt.

It is the object of the present invention to provide a process for the preparation of 1-phenyl-3-(4-chlorophenyl)-pyrazol-4-ylacetic acid and its calcium salt in high yields and high purity without use of expensive reactants.

The avobe object is achieved according to claim 1. The dependent claims relate to preferred embodiments.

According to the invention, the avobe acid can be advantageously obtained in good yields and high purity by reductive hydrolysis of 1-phenyl-3-(4-chlorophenyl)-4-pyrazolecarboxaldehyde cyanohydrine with at least an equivalent amount of stannous chloride in a mixture of acetic acid and hydrochloric acid. The reaction is carried out at an elevated temperature substantially within the range of from 60 °C to the boiling temperature of the reaction mixture, preferably in the presence of a catalytic amount of hydroiodic acid, which is beneficial as regards the purity of the desired product. The starting cyanohydrine is obtained using liquid or nascent hydrogen cyanide, conveniently from sodium or potassium cyanide and a weak acid in a lower alcohol; the crude wet material can advantageously be used as such since moisture does not interfere and the unreacted aldehyde if present is easily separated and re-used. The resulting free acid is suitably neutralized in situ with an aqueous alkali, and calcium salt, if required is precipitated with a solution of calcium chloride.

In the following, the method according to the invention will be explained with reference to examples.

## Example 1

1-Phenyl-3-(4-chlorophenyl)-4-pyrazolecarboxaldehyde cyanohydrine

To a mixture of 8,3 g (0,167 mole) of sodium cyanide and 4 ml of water there are added 28,3 g (0,1 mole) of 1-phenyl-3-(4-chlorophenyl)-4-pyrazolecarboxaldehyde and 200 ml of methanol with stirring at 25 °C. Stirring is continued for 5 min; thereafter 9,5 ml of acetic acid are added within 5 min, and the heterogeneous mixture is then stirred for 1,25 h at 25 to 30 °C, whereby a transient dissolution of the solids and subsequent spontaneous thickening of the mixture occur. The reaction mixture is diluted with 300 ml of water, and after 5 min stirring the precipitate is collected on a sintered glass filter and washed with 2 x 30 ml of water to give 66 to 80 g of wet product containing, depending on suction efficiency, 35 to 49 g of water; the filter cake can be immediately, without drying or purification, used as such for subsequent reductive hydrolysis with stannous chloride in an aqueous acidic medium. Free drying of the wet material in the air affords 31 g, i.e. in quantitative yield, of a product consisting, after HPLC analysis, of 91% of the desired cyanohydrine, 6 % of the unreacted aldehyde and 3 % of not identified impurities. Crystallization from methanol or ethanol yields 25,4 g (82,1 % yield) of 1-phenyl-3-(4-chlorophenyl)-4-pyrazolecarboxaldehyde cyanohydrine; m.p. 124 to 126,5 °C; purity 99 % (HPLC).

## Example 2

1-Phenyl-3-(4-chlorophenyl)-pyrazol-4-ylacetic acid

To a mixture of 31,0 g (0,1 mole) of the preceding cyanohydrine, 60 ml of acetic acid, 60 ml of concentrated hydrochloric acid and 0,5 ml of concentrated hydroiodic acid there are added 24,0 g (0,106 mole) of stannous chloride dihydrate; the mixture is warmed gently to boiling and refluxed for 2 h. The reaction mixture is then cooled to 25 °C and filtered and the solids are washed on the filter with 10 ml of acetic acid. The combined filtrates are diluted with water (250 ml) and extracted twice with chloroform (250 and 50 ml). The combined organic phases are washed with water (100 ml) and evaporated to dryness on a vacuum rotary evaporator. The solid residue is then crystallized from aqueous ethanol or methanol to yield 30 g (95.9 % yield) of 1-phenyl-3-(4-chlorophenyl)-pyrazol-4-ylacetic acid; m.p. 151 to 151,5 °C; purity 99,94 % (HPLC).

Example 3

1-Phenyl-3-(4-chlorophenyl)-pyrazol-4-ylacetic acid (from crude wet cyanohydrine)

To a mixture of 66 g of wet cyanohydrine filter cake (moisture content 35,2 g, dry matter composition after HPLC: 91 % of 1-phenyl-3-(4-chlorophenyl)-4-pyrazolecarboxaldehyde cyanohydrine, 6,5 % of unreacted starting aldehyde and 2.5 % of impurities), 115 ml of acetic acid and 70 ml of concentrated hydrochloric acid there are added 24 g (0,106 mole) of stannous chloride dihydrate, and the resulting mixture is heated for 3 h at 110 °C. The reaction mixture is then cooled to 25 °C, filtered and distributed between 225 ml of chloroform and 250 ml of water. The aqueous phase is separated and extracted with 40 ml of chloroform. the combined organic phases are washed twice with water (100 and 80 ml) and thereafter extracted with 250 ml of 1 N aqueous potassium hydroxide solution. The organic phase is separated and extracted with 50 ml of 0,25 N potassium hydroxide solution. The aqueous alkaline extracts are combined, then 200 ml of chloroform are added, and the extracts are adjusted with hydrochloric acid to pH 3. After shaking the chloroform layer is separated, evaporated to a final volume of 60 ml, cooled to 15 °C and, if necessary inoculated. After 3 h of standing the formed crystals are collected on a sintered glass filter and washed with precooled chloroform to give 25,8 g (82,5 % yield) of 1-phenyl-3-(4-chlorophenyl)-pyrazol-4-ylacetic acid having the same properties as the product of the preceding example.

Example 4

Calcium 1-phenyl-3-(4-chlorophenyl)-pyrazol-4-ylacetate

To a mixture of 66 g of wet cyanohydrine (with the same moisture content and dry matter composition as the starting material of example 3) and 120 ml of acetic acid there are added 60 ml of concentrated hydrochloric acid, 0,5 ml of concentrated hydroiodic acid and 24 g (0,106 mole) of stannous chloride dihydrate. The whole resulting mixture is heated with stirring within 0,5 h to the boiling temperature, whereby the solids dissolve. After 2 h of continuous stirring under gentle reflux the reaction mixture is cooled, and the solids are separated and washed with 10 ml of acetic acid. The combined acidic filtrates are shaken thoroughly with 250 ml of chloroform and 250 ml of water; the aqueous phase is separated and extracted with 40 ml of chloroform. The organic phases are combined, washed twice with water (100 and 50 ml) and thereafter extracted successively with 250 ml of 1 N sodium hydroxide solution and 40 ml of 0,5 N sodium hydroxide solution. The aqueous alkaline phases are combined, extracted twice with chloroform (100 and 60 ml), warmed for 10 min to the boiling temperature or bubbled with nitrogen at 70 °C until complete removal of chloroform and then filtered. The filtrate is admixed under stirring For 5 min at 70 °C with a filtered solution of 0,15 mole of calcium chloride (anhydrous, dihydrate or hexahydrate) in 180 ml of water. The suspension is cooled to 20 °C and after 30 min filtered through a sintered glass filter. The separated solid product is washed with 4 x 70 ml of water und dried at 100 °C. The yield is 28,6 g (86,1 %) of calcium 1-phenyl-3-(4-chlorophenyl)-pyrazol-4-ylacetate; m.p. 258 to 262 °C; purity 99.86 % (HPLC). The combined chloroform phases are evaporated to dryness on a vacuum rotary evaporator, and the residue is crystallized from methanol, ethanol or acetone. Their resulting 1,5 g (5,1 % yield of crystalline 1-phenyl-3-(4-chlorophenyl)-4-pyrazolecarboxaldehyde (m.p. 141 to 143 °C) is re-used for the preparation of the starting cyanohydrine.

Example 5

The procedure of example 4 is repeated, with the only difference that the reductive hydrolysis with stannous chloride is carried out at 60 °C for 8 h. The calcium salt is obtained in a yield of 81,7 %; purity 99,84 %.

## Example 6

The procedure of example 4 is repeated, with the difference that a cyanohydrine of 99,0 % purity and 1,2 equivalents of stannous chloride are used for the reductive hydrolysis. The calcium salt is obtained in a yield of 94,9 %.

## Example 7

The procedure of example 4 is repeated, with the difference that the reductive hydrolysis with stannous chloride is carried out with use of a stoichiometric amount of the cyanohydrine (99 % purity), and the salt formation using calcium chloride is conducted at 90 °C. The calcium salt is obtained in a yield of 94,3 %.

## Example 8

The procedure of example 4 is repeated, with the difference that hydroiodic acid is used in a twofold or half the amount, i.e. 1,0 or 0,25 ml respectively, and diethyl ether, dichloromethane or dichloroethane are used in place of chloroform for extractions. The calcium salt is obtained in a yield of 86,0 % (99.85 % purity).

## Example 9

The procedure of example 4 is repeated, with the difference that 0,125 mole of calcium chloride is used. The desired calcium salt is obtained in a yield of 78 %.

## Example 10

The procedure of example 4 is repeated, with the difference that the salt formation using calcium chloride is carried out at 10 °C. The calcium salt is obtained in a yield of 86,2 % (purity 99,85 %).

## Claims

1. A process for the preparation of 1-phenyl-3-(4-chlorophenyl)-pyrazol-4-ylacetic acid and its calcium salt, **characterized** by reductive hydrolysis of 1-phenyl-3-(4-chlorophenyl)-4-pyrazolecarboxaldehyde cyanohydrine with at least an equivalent amount of stannous chloride in a mixture of acetic acid and hydrochloric acid at temperatures substantially within the range of from 60 °C to the boiling temperature of the reaction mixture and, if required, subsequent conversion of the acid into its calcium salt.

2. The process according to claim 1, characterized in that the reductive hydrolysis is conducted in the presence of a catalytic amount of hydroiodic acid.

3. The process according to claim 1 or 2, characterized in that the formed 1-phenyl-3-(4-chlorophenyl)-pyrazol-4-ylacetic acid is neutralized in situ with an aqueous alkali, and the required calcium salt is precipitated with a calcium chloride solution.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 320 095 (SCHERING) <br> * Whole document * <br> --- | | C 07 D 231/12 |
| A | AU-A- 514 889 (SCHERING) <br> * Whole document * <br> --- | | |
| A | EP-A-0 022 906 (SCHERING) <br> * Whole document * <br> --- | | |
| A | US-A-4 146 721 (RAINER) <br> * Whole document * <br> --- | | |
| A | FR-A-2 133 503 (FUVEAU) <br> * Whole document * <br> --- | | |
| A | FR-A-2 122 333 (FUVEAU) <br> * Whole document · * <br> --- | | |
| A | EP-A-0 094 555 (BYK GULDEN) <br> * Whole document * <br> ----- | | |

**TECHNICAL FIELDS SEARCHED (Int. Cl.4)**

C 07 D 231/00

The present search report has been drawn up for all claims

| Place of search | Date of completion of the search | Examiner |
|---|---|---|
| THE HAGUE | 12-10-1988 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document